# EUROPEAN PATENT APPLICATION

(11) **EP 4 763 481 A1**
(43) Date of publication of application: **24.06.2026**
(21) Application number: 24383455.3
(22) Date of filing: 23.12.2024
(51) Int. Cl.: B29C 64/135, B29C 64/245, B29C 64/255, B33Y 10/00, B33Y 30/00, B29C 64/227, A61F 2/82, B33Y 80/00, B29D 23/00

(54) **3D PRINTING DEVICE FOR MANUFACTURING TUBULAR 3D OBJECTS, CORRESPONDING PRINTING METHOD AND OBJECT**

(71) Applicant: Fundació Eurecat, 08290 Cerdanyola del Vallès (ES)
(72) Inventor: Bosch i Collell, Aniol, 17160 Anglès (ES); Guerra Sánchez, Antonio Jesús, 17190 Salt (ES)
(74) Representative: Curell Suñol S.L.P.

(57) **Abstract**

3D printing device (1) for manufacturing tubular 3D objects (2) by 3D printing according to printing models; said device (1) comprising a build platform (3) having a tubular shaped build surface (4), and rotatably mounted around a horizontal axis; a rotation control means (7), configured to control the rotation of said build platform (3); a laser source (10); a beam positioning means (11), configured to variably direct the laser beam; a plurality of resin tanks (51) and at least one cleaning tank (52); a tank positioning means, configured to change the relative position between one tank and said build platform (3) to a usage position wherein said tank is under said build platform (3); and a control means, configured to control the different elements according to a printing model to manufacture a 3D object (2).

## Description

### Field of the invention

The invention refers to the field of manufacturing objects by means of additive manufacturing processes, generally known as 3D printing.

In particular, the invention relates to a 3D printing device, for manufacturing at least one tubular 3D object, in particular a stent, each 3D object manufactured by 3D printing according to a printing model; said device comprising:
- a build platform having a tubular shaped build surface, said build platform rotatably mounted around a longitudinal axis arranged horizontally;
- a rotation control means, configured to control the rotation of said build platform around said longitudinal axis;
- a laser source configured to emit a laser beam; and
- a beam positioning means, configured to variably direct said laser beam towards different points of said build surface.

The invention also relates to a 3D printing method for manufacturing at least one tubular 3D object, and a tubular 3D object obtainable by said 3D printing method.

### Prior art

3D printing of tubular objects is known in the art, for example, WO/2022/090451 A1 discloses a printing device based on stereolithography over a tubular build platform to manufacture by 3D printing a tubular 3D object with a high degree of precision, among other advantageous features.

Multi-material objects may be particularly useful in some situations. As a non-limiting example, in the case where the 3D tubular object is a stent for medical use it may be advantageous that the stent itself includes both a structural part that remains stable with time and a medical treatment agent that dissolves with time, thereby slowly liberating said treatment agent into the body of the patient. In this case, the multiple parts are aimed to different objectives so that multiple materials must be used when manufacturing the stent.

Some solutions in the art have considered the creation of multi-material 3D printed tubular objects. For example, manufacturing different parts of the object separately, the object having a mechanical assembly design wherein the different parts can be engaged together in a single object. However, this type of solutions substantively limits the possible object designs and also renders the overall manufacturing process complex and difficult. Moreover, it may be difficult to use this type of solutions for stents of the type mentioned above because the structural parts must remain stable.

Therefore, it is necessary to provide a solution for manufacturing 3D printed tubular objects that can be made of multiple materials, and wherein, additionally, the manufacturing process remains as simple as possible.

### Summary of the invention

The invention is aimed to provide a 3D printing device of the type stated at the beginning, being able to avoid the problems that have been identified above.

This purpose is achieved by a 3D printing device of the type indicated at the beginning, characterised in that said device further comprises:
- a plurality of tanks, comprising a plurality of resin tanks, each resin tank configured for containing a fluid resin of a type that can be polymerised by said laser, and at least one cleaning tank, each cleaning tank configured for containing a cleaning product;
- a tank positioning means, configured to change the relative position between one tank selected among said plurality of tanks and said build platform to a usage position wherein said tank is under said build platform, such that said fluid resin or said cleaning product of said tank contacts said build surface of said build platform; and
- a control means, configured to control said laser source, said beam positioning means, said rotation control means, and said tank positioning means, according to said printing model to manufacture said 3D object.

The 3D printing device uses a tubular shaped build surface that is arranged horizontally and rotates around an axis in order to convey the resin to the zones wherein the laser beam impinges the surface, so that the resin is polymerised in precise points by the precise positioning the laser beam and the rotation of the build platform. The basis of this type of solution is known from WO/2022/090451 A1 so, for the sake of brevity, it is only briefly described here. However, contrary to the known solutions, the invention disclosed herein also comprises a plurality of tanks, each tank aimed to contain either a laser-polymerizable resin or a cleaning product, for example, a cleaning liquid. The tank positioning means changes the relative position between one of the tanks and the build platform to a usage position. Said change in the relative position may be achieved, for example, by changing the tank position or by changing the build platform position. In the usage position the tank and build platform are arranged so that the resin or cleaning product passes from the tank to the build surface, in particular, directly over said surface or over previous layers of resin that have already been polymerized. For example, in the case of a resin, it may be envisaged that the build platform is partially submerged in the resin tank, then, the rotation control means drive the build platform to rotate. The rotation speed is selected based on the resin viscosity, such that the rotation of the platform generates a dragging effect that extends the resin over the build surface and, in particular, over the required zone that can then be impinged by the laser beam. Those skilled in the art will have no problems in selecting the particular rotation speed using the criteria discussed herein. A similar strategy may be envisaged for the cleaning products, however, other possibilities are disclosed below. The 3D printing device is able to perform multiple printing stages, firstly with an initial resin, and later on with one or more other resins. The objective of the cleaning products is cleaning up the resin residues between stages and/or performing a final cleaning for the printed object. Depending on the resins, it may be necessary to use more than one cleaning product. Embodiments may be envisaged wherein the resin tanks and cleaning tanks are equal but, depending on the characteristics of the resins and cleaning product(s) they may also be envisaged to have different forms.

Therefore, the 3D printing device disclosed herein allows the manufacture of 3D objects having a tubular structure and made of more than one type of resin. Moreover, it allows the precise positioning of the layers of each type of resin since the object is not only not removed from the build platform, but it is not even removed from the printing device. This technical effect solves the problem of precise alignment of layers of different materials, and their interconnections. Using the same process for printing all the resins before curation and without any required assembly also has the technical effect that the layers of different resins may attach or weld to one another by the resin polymerisation itself, without the need for particular layer shapes, glue or any other type of supporting elements. The resulting 3D printed object is therefore more stable, secure and easy to manufacture, and may have a lower profile than other solutions known in the art. In addition, the printing device of the invention allows to manufacture the complete object with all the required layers, without the explicit need for a UV curation between each layer.

Preferably, said control means is configured for performing at least one printing sequence comprising the following steps:
- positioning a resin tank selected among said plurality of resin tanks under said build platform in said usage position;
- controlling said rotation control means, said laser source and said beam positioning means to polymerize said fluid resin coming from said resin tank according to said printing model.

Those skilled in the art will understand that the sequence described above may also comprise further steps, and the overall printing process may comprise several sequences to manufacture a 3D object made of different materials.

Preferably, said control means is further configured for performing a cleaning sequence after at least one of said printing sequences, which is an advantageous option, in particular if the resin of two successive sequences is different. A final cleaning sequence after all the printing sequences may also be preferably envisaged.

Preferably, said control means is further configured for performing a cleaning sequence between each two successive printing sequences, more preferably, only in the case that the fluid resin contained in the resin tank selected in a first printing sequence of said two successive printing sequences is different from the resin contained in the resin tank selected in a second printing sequence of said two successive printing sequences. Therefore, the excess of resin can be cleaned before the next layer, which is particularly advantageous in the case where two consecutive layers are made of different resins.

Preferably, said tank positioning means is configured to change the position of said plurality of tanks. As a first option, the relative position between the tank and the build platform used to arrange one tank selected among the plurality of tanks to the usage position is done by moving the position of the tank, thereby allowing that said build platform remains in the same position when said selected tank is placed under said build platform in said usage position. This option has the advantage that it is not necessary to move the build platform or any other structural element, thereby minimizing the possibility of misalignments and granting the structural integrity of said elements. The drawback is that caution must be exercised when moving the tanks so that their contains do not spill outside.

Preferably, said tank positioning means comprises a sliding cart, wherein said plurality of resin tanks and said at least one cleaning tank are attached to said sliding cart, such that positioning a resin tank selected among said plurality of resin tanks, or a cleaning tank of said at least one cleaning tank, in a usage position under said build platform is done by linearly sliding said sliding cart, and then raising said resin tank or said cleaning tank to said usage position. This embodiment facilitates the manufacture and design of the 3D printing device. In a preferred embodiment, the sliding cart is arranged movably in a horizontal direction perpendicular to the longitudinal axis of the build platform.

Preferably, said tank positioning means comprises a carrousel rotatably mounted around a vertical axis, wherein said plurality of resin tanks and said at least one cleaning tank are attached to said carrousel, such that positioning a resin tank selected among said plurality of resin tanks, or a cleaning tank of said at least one cleaning tank, in a usage position under said build platform is done by rotating said carrousel and then raising said resin tank or said cleaning tank to said usage position. This option of moving the tanks has the advantage of minimizing the space required for the overall 3D printing device, which is particularly well suited for using said device in places where the room space is tight.

Preferably, said tank positioning means is configured to change the position of said build platform, such that said plurality of tanks can remain in the same position when the build platform is placed over said tank selected among said plurality of tanks in said usage position. An alternative of the relative positioning can be obtained by moving the build platform and possibly the related supporting structures. The main advantage of not moving the tanks is that the fluid resin or cleaning product remains also static without agitation and avoiding undesired effects like creating waves or bubbles.

Preferably, said tank positioning means further comprises tank elevating means, configured to raise said tank positioned under said build platform to said usage position and to lower said resin tank or said cleaning tank from said usage position to a free position wherein said resin tank or said cleaning tank does not contact said build platform and can be moved away from said build platform. This option may be compatible in the cases where the tanks are moved, and also in the cases where the build platform is the part that is moved.

Preferably, said tank elevating means comprises a vertical guide for each resin tank of said plurality of resin tanks and each cleaning tank of said at least one cleaning tank, configured to allow a vertical movement of said resin tank and said cleaning tank while preventing horizontal displacement; said vertical guide preferably comprising at least two parallel pillars, each pillar inserted in a corresponding guiding hole fixed to said resin tank or said cleaning tank. The vertical guide helps stabilizing the vertical movement and aligning the relative position of the tank and the build platform in the usage position. The option with two parallel pillars facilitates the design of the device and minimizes the cost, more parallel pillars may be envisaged, for example, four pillars one in each corner of the tank.

Preferably, said tank elevating means further comprises at least one cam configured to push said resin tank or said cleaning tank in a vertical direction, so that the elevation may be controlled by a rotating element like a small electrical motor, for example.

Preferably, said at least one cam is arranged under said build platform, such that the same cam is used for each of said resin tanks and each of said cleaning tanks. Therefore, the same cam is used for all the tanks, which minimizes the number of elements required in the 3D printing device.

Preferably, said at least one cam comprises a plurality of cams, one cam arranged under each resin tank of said plurality of resin tanks and one cam arranged under each cleaning tank of said at least one cleaning tank. Therefore, each tank is associated with its own cam. This option may be particularly advantageous to cope with tanks with different characteristics and dimensions.

Preferably, at least one of said cleaning tanks and more preferably each of said cleaning tanks is provided with an idle roller horizontally arranged over a cleaning product repository, wherein, in said usage position, said idle roller is parallel to said build platform and contacts said build surface of said build platform, such that a rotation of said build platform drives said idle roller to rotate and said cleaning product is transferred from said repository to said build surface by means of said idle roller. This option is particularly well suited for cleaning the residues of resin, the idle roller not only conveys the cleaning product to the build surface and the layers of the object that is being printed, but also contributes to drag the non-polymerized resin out of the build surface.

Preferably, the device further comprises resin temperature control means that are configured to control, at least, the temperature of a resin contained in one resin tank of said plurality of resin tanks, preferably at least when said resin tank is in said usage position. It has been found that controlling the temperature of the resin it is possible to control its viscosity and, even more importantly, the thickness of the resin layer that is arranged over the build surface. Therefore, temperature control means allows for a precise control of the thickness of the resin layer. Temperature control means may not always be necessary, for example, it is possible that some resins are not suitable for this change of thickness/viscosity by controlling the temperature, or it may also be envisaged that the room temperature may be controlled for all the resins. However, having the possibility of precise temperature control resin-by-resin gives the device a great versatility to adapt to multiple resins and their combinations. The particular option of controlling only the tank that is in usage position minimizes the number of elements required for the device, but it may increment the build time because the resin has to be driven to the required temperature only after the tank is positioned. This may be solved by embodiments wherein said resin temperature control means is configured to control the temperature of each resin contained in each resin tank of said plurality of resin tanks.

Preferably, said resin temperature control means comprises heating means configured for heating the resin. The heating means may preferably comprise at least one resistance and/or a thermoelectric cell, also known as a Peltier cell.

Preferably, said resin temperature control means comprises cooling means configured for cooling the resin. The cooling means may preferably comprise at least one thermoelectric cell. In particular, said at least one thermoelectric cell may be used as cooling means and also as heating means.

The invention also refers to a 3D printing method for manufacturing at least one tubular 3D object, in particular a stent, each 3D object manufactured by 3D printing according to a printing model; said method making use of a 3D printing device according to any one of the embodiments disclosed above, wherein said method is executed by said control means by controlling said laser source, said beam positioning means, said rotation control means, and said tank positioning means according to said printing model.

The invention also refers to a tubular 3D object obtainable by the 3D printing method as described above, wherein said object is made of at least two different resins, and wherein, in at least one point of said object, at least two of said different resins are welded together forming radial layers, wherein a layer made of a first resin is under a layer made of a second resin, and wherein said layers are welded by the polymerization of said resins. Preferably, said object is a stent.

The invention also includes other detail features illustrated in the detailed description of some embodiments of the invention and in the accompanying figures.

### Brief description of the figures

Further advantages and features of the invention will become apparent from the following description, in which, without any limiting character, preferred embodiments of the invention are disclosed, in reference to the accompanying figures:
Fig. 1 is a perspective view of a 3D printing device according to one embodiment of the invention.
Fig. 2 is a perspective detail view of the 3D printing device of Fig. 1.
Fig. 3 is a top view of the detail of Fig. 2.
Fig. 4 is a front view of the detail of Fig. 2 and Fig. 3.
Fig. 5 is a perspective detail view of the carrousel and cam of the 3D printing device of Fig. 1.
Fig. 6 is a perspective detail view of a 3D printing device according to another embodiment of the invention.
Fig. 7 is a schematic view of a stent printed over the build surface of a 3D printing device according to the invention.
Fig. 8 is a schematic view of the stent of Fig. 7 once removed from the build platform.
Fig. 9 is a schematic perspective view of a 3D printing device according to another embodiment of the invention.

### Detailed description of embodiments of the invention

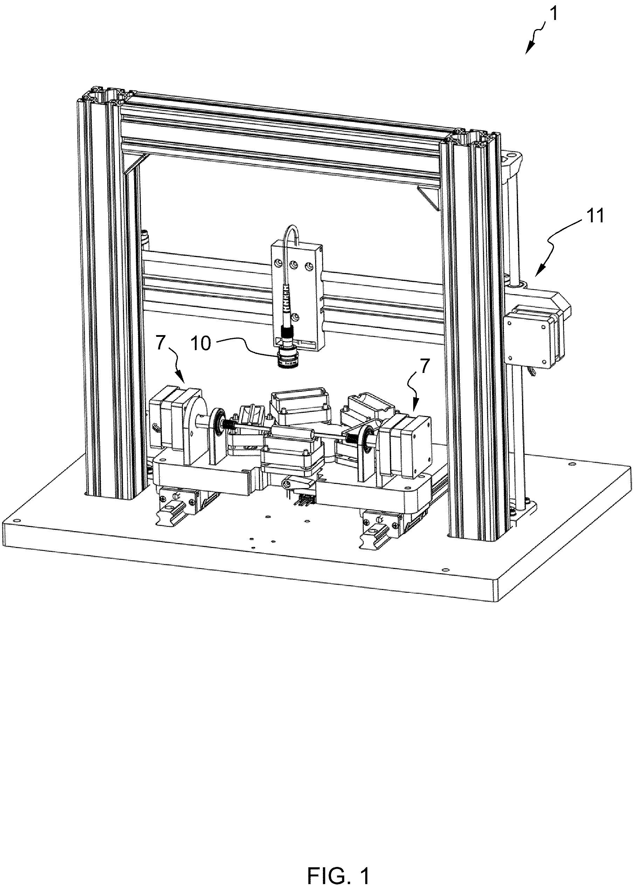

Fig. 1 to Fig. 5 show a first embodiment of a 3D printing device 1 according to the invention. The device 1 is aimed for manufacturing tubular 3D objects 2 by 3D printing according to the corresponding printing models. The device 1 is aimed, in particular, for manufacturing stents, and more in particular, stents made of multiple materials.

The device 1 of the first embodiment is provided with a build platform 3 that has a tubular shaped build surface 4, in particular having a cylindrical shape. Said build surface 4 is where the 3D objects are built using light-polymerizable resins. The build platform 3 is rotatably mounted around a longitudinal axis that is arranged horizontally. The rotation of the build platform 3 around said longitudinal axis is controlled by a rotation control means 7. Said rotation control means 7 are used for positioning the build platform 3 in a specific position, but also for spinning the build platform 3 in order to distribute the resin or cleaning product over the build surface 4.

The device 1 is also provided with a source of electromagnetic radiation, in particular, a laser source 10 that emits a laser beam. A beam positioning means 11 is provided and used to variably direct said laser beam towards different points of the build surface 4, such that the laser impinges in the resin that has been transported over the build platform or in a previous resin layer of the 3D object that its being built. In the embodiment, said beam positioning means 11 comprise a heigh control that moves the horizontal bar shown in Fig.1 up and down, and thereby controls the focus of the laser beam. Additionally, the beam positioning means 11 also control the horizontal position of the laser source 10 in said horizontal bar. The laser source 10 is arranged to emit the laser beam downwards, such that said laser beam is directed towards the centre of the highest point of the build surface 4. Therefore, by controlling the rotational position of the build platform 3 and the horizontal position of the laser source 10 it is possible to precisely control where the resin has to be polymerized according to the printing model. With the height position of the laser source 10 it is possible to control the focus of the laser beam and, therefore, the size of the spot that will be polymerized.

The device 1 further comprises a plurality of tanks 5. Among said tanks there is a plurality of resin tanks 51 and at least one cleaning tank 52. All the five tanks 5 in the Fig. 1 to Fig. 5 are equal, so that at least one is deemed to be a cleaning tank 52 and the remaining ones are deemed to be resin tanks 51. Other embodiments may be envisaged wherein resin tanks 51 are different from cleaning tanks 52. Each resin tank 51 contains a fluid resin of a type that can be polymerised by the laser beam. Each cleaning tank 52 is configured for containing a cleaning product aimed for cleaning up the remains of resin over the build surface. In general, the determination of which of the tanks 5 contains which particular fluid resin and which of the tanks contains a cleaning product is done before starting printing a particular object, so that the sequence of layers can be pre-programed. The device 1 is therefore suitable for printing with different resins using different resin tanks 51. It may also be envisaged that the same fluid resin is provided in two or more resin tanks 51, for example, if the number of layers of said resin to be polymerised is large.

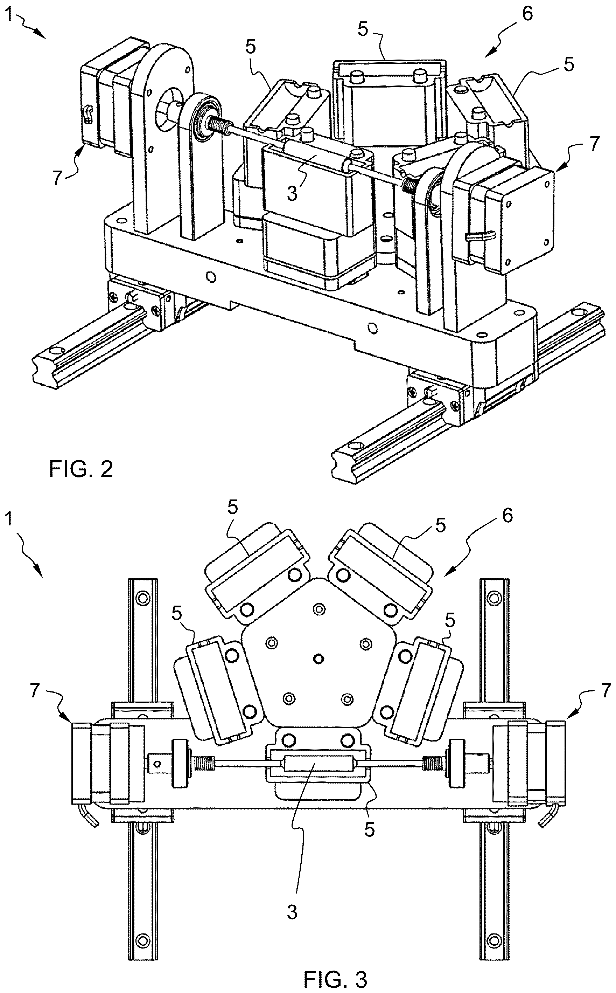

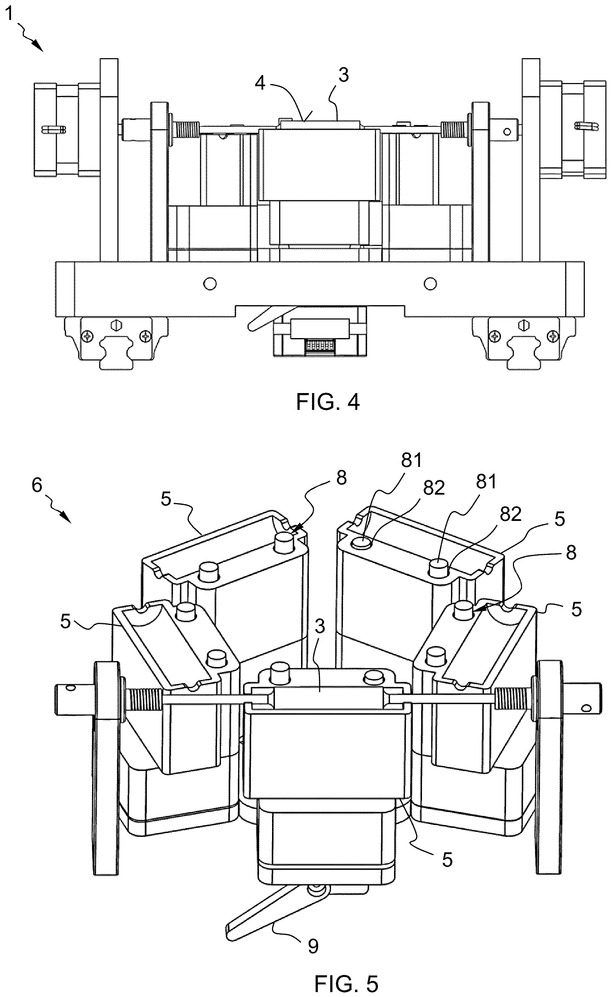

Fig. 2 and Fig. 3 show that the device 1 is further provided with a tank positioning means that are configured to change the relative position between one tank 5 selected among the plurality of tanks 5 and the build platform 3 to a usage position wherein said selected tank 5 is under said build platform 3, such that the fluid resin or the cleaning product contained in the selected tank 5 contacts the build surface 4 of the build platform 3. In the first embodiment, the tank positioning means is configured to change the position of said plurality of tanks 5. In particular, a carrousel 6 is rotatably mounted around a vertical axis, and all the tanks 5 are attached to said carrousel 6. This way, in order to position the selected tank 5 in the usage position, the carrousel 6 is rotated such that the corresponding tank 5 is positioned under the build platform 3, and then the tank 5 is raised to the usage position. Lowering and raising the selected tank 5 from the usage position to a free position wherein the tank 5 does not contact the build platform 3 and can be moved away from it (and vice-versa) is done by means of tank elevating means. Said tank elevating means comprise a vertical guide 8 for each tank 5 that is configured to allow a vertical movement of the tank 5 while preventing horizontal displacement. As shown in Fig. 5, in the first embodiment the tank elevating means comprise two parallel pillars 81, each pillar 81 inserted in a corresponding guiding hole 82 fixed to the tank 5. One cam 11 is arranged under the build platform 3 and used for raising and lowering the selected tank 5. The cam 11 is used for all of the tanks 5, however, other embodiments may be envisaged wherein each tank 5 is provided with its own corresponding cam 11

The laser source 10, beam positioning means 11, rotation control means 7, and tank positioning means, are controlled by a control means (not shown in the figures), in order to manufacture a 3D object 2 according to a particular printing model. The object 2 is manufactured by printing consecutive concentric layers forming a tubular structure. The control means is configured for performing one or more printing sequences, depending on the 3D object 2 to be manufactured. Each printing sequence has the following steps:
- positioning a resin tank 51 selected among said plurality of resin tanks 51 under said build platform 3 in the usage position;
- controlling said rotation control means 7, said laser source 10 and said beam positioning means 11 to polymerize said fluid resin coming from said resin tank 51 according to said printing model.

Cleaning sequences are also used when printing, in particular between two consecutive printing sequences that use different resins. A final cleaning sequence may also be envisaged.

When a resin tank 51 is positioned in the usage position, the control means use the rotation control means 7 in order to drive the build platform 3 to spin for several rounds and at a pre-defined speed in order to adequately cover the build surface with the resin. The required number of rounds and speed depend on the characteristics of the resin and, in particular, to its viscosity. The viscosity also affects to the thickness of the layer of resin that will be polymerized.

In order to control the viscosity of a resin, the device 1 of the first embodiment further comprises resin temperature control means for each of the resin tanks 51. Such temperature control may also be obtained, for example, by controlling the environmental temperature of the location where the device 1 is being used. However, by adding temperature control means for each tank 5, the viscosity of each particular resin can be precisely controlled. In the first embodiment, the temperature control means is a thermoelectric cell, also known as Peltier cell, so that temperature can be increased (heating) or decreased (cooling). Other temperature control means may be envisaged, for example, using resistors for heating.

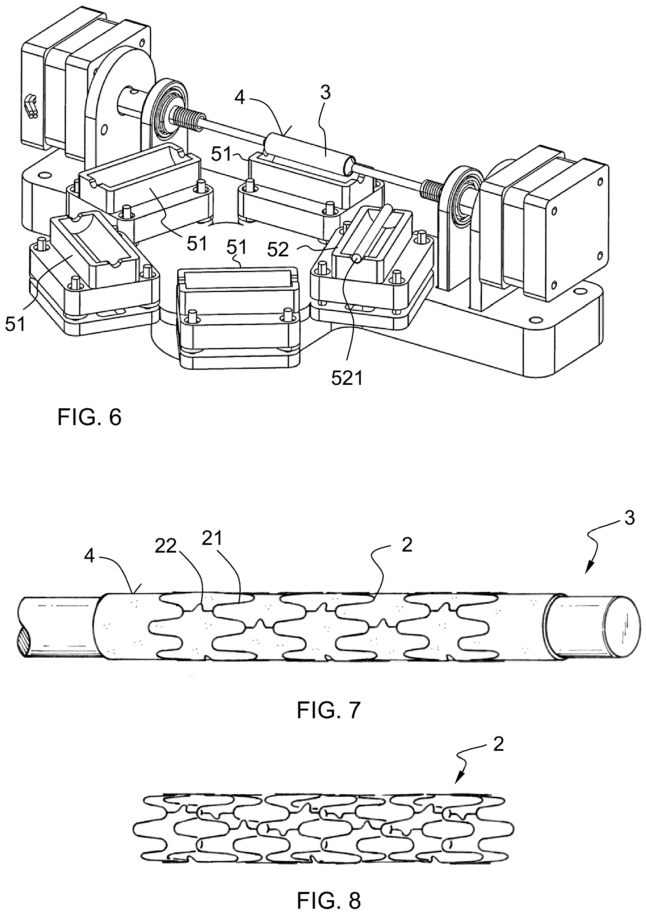

Fig. 7 and Fig. 8 show a tubular 3D object 2 that has been obtained with the 3D printing device 1 performing the corresponding printing method. The example corresponds to a stent. Fig. 7 shows the object 2 still attached to the build platform 3 as it has been printed over the build surface 4, and Fig. 8 shows the object 2 once it has been removed from the build platform 3. The object 2 is a non-limiting example formed by two layers of different resins, a first layer 21 formed by a first printing sequence wherein different wavy concentrical patterns are printed using a first resin, and a second layer 22 formed by a second printing sequence wherein different bridges are formed between the wavy concentrical patterns using a second resin. The first and second layers 21, 22 are welded together by the polymerization of the resins, and not by another type of bonding.

Other embodiments of the 3D printing device according to the invention are disclosed hereinafter. These embodiments share most of the features disclosed in the first embodiment above. Therefore, only the differentiating features will be described in detail. For the sake of brevity, common features shared with the first embodiment disclosed above will not be described again hereinbelow.

Fig. 6 shows a detail of a second embodiment of the device 1 according to the invention, wherein one cleaning tank 52 is provided with an idle roller 521 that is horizontally arranged over a cleaning product repository. In the usage position, the idle roller 521 is parallel to the build platform 3 and contacts said build surface 4, such that a rotation of said build platform 3 drives said idle roller 521 to rotate and the cleaning product is transferred from the repository to said build surface 4 by means of the idle roller 521.

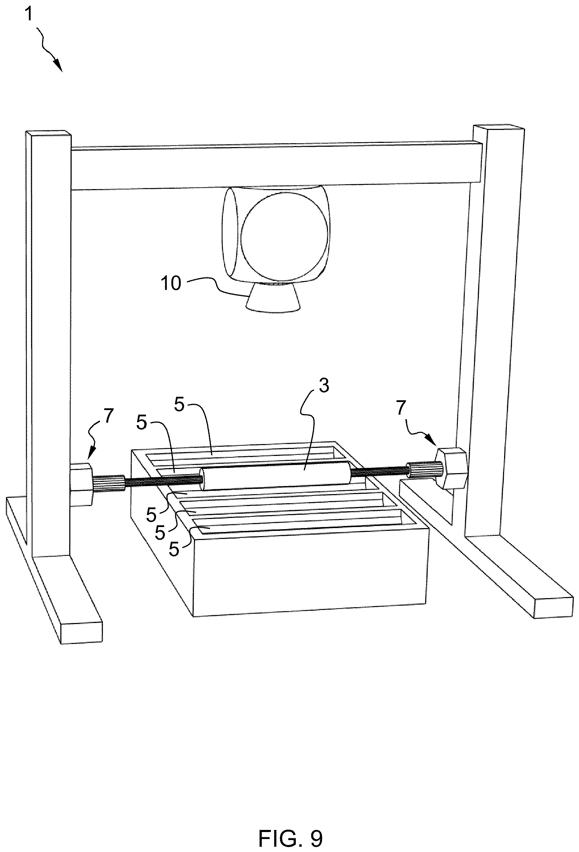

Fig. 9 shows a third embodiment of a device 1 according to the invention. The figure is a schematic drawing aimed for representing the main components, not to be an accurate depiction of the device. In this embodiment, the tank positioning means comprises a sliding cart 12 instead of a carrousel 6. The plurality of tanks 5 are attached to said sliding cart 12 or formed in said sliding cart 12. Therefore, positioning a tank 5 selected among the plurality of tanks 5 in a usage position under the build platform 3 is done by linearly sliding the sliding cart 12 (in this case, in a horizontal direction perpendicular to the longitudinal axis of the build platform), and then raising said tank 5 or the complete sliding cart 12 to the usage position.

In a fourth embodiment not shown in the figures, the tank positioning means is configured to change the position of said build platform 3 instead of the position of the tanks.

## Claims

1. 3D printing device (1), for manufacturing at least one tubular 3D object (2), in particular a stent, each 3D object (2) manufactured by 3D printing according to a printing model; said device (1) comprising:
- a build platform (3) having a tubular shaped build surface (4), said build platform (3) rotatably mounted around a longitudinal axis arranged horizontally;
- a rotation control means (7), configured to control the rotation of said build platform (3) around said longitudinal axis;
- a laser source (10) configured to emit a laser beam; and
- a beam positioning means (11), configured to variably direct said laser beam towards different points of said build surface (4);
**characterised in that** said device (1) further comprises:
- a plurality of tanks (5), comprising a plurality of resin tanks (51), each resin tank (51) configured for containing a fluid resin of a type that can be polymerised by said laser beam, and at least one cleaning tank (52), each cleaning tank (52) configured for containing a cleaning product;
- a tank positioning means, configured to change the relative position between one tank (5) selected among said plurality of tanks (5) and said build platform (3) to a usage position wherein said tank (5) is under said build platform (3), such that said fluid resin or said cleaning product of said tank (5) contacts said build surface (4) of said build platform (3); and
- a control means, configured to control said laser source (10), said beam positioning means (11), said rotation control means (7), and said tank positioning means, according to said printing model to manufacture said 3D object (2).

2. Device (1) according to claim 1, wherein, said control means is configured for performing at least one printing sequence comprising the following steps:
- positioning a resin tank (51) selected among said plurality of resin tanks (51) under said build platform (3) in said usage position;
- controlling said rotation control means (7), said laser source (10) and said beam positioning means (11) to polymerize said fluid resin coming from said resin tank (51) according to said printing model.

3. Device (1) according to claim 2, wherein, said control means is further configured for performing a cleaning sequence after at least one of said printing sequences.

4. Device (1) according to any one of the claims 1 to 3, wherein said tank positioning means is configured to change the position of said plurality of tanks (5).

5. Device (1) according to claim 4, wherein said tank positioning means comprises a sliding cart (12), wherein said plurality of tanks (5) are attached to said sliding cart (12), such that positioning a tank (5) selected among said plurality of tanks (5) in a usage position under said build platform (3) is done by linearly sliding said sliding cart (12), preferably in a horizontal direction perpendicular to said longitudinal axis, and then raising said tank (5) to said usage position.

6. Device (1) according to claim 4, wherein said tank positioning means comprises a carrousel (6) rotatably mounted around a vertical axis, wherein said plurality of tanks (5) are attached to said carrousel (6), such that positioning a tank (5) selected among said plurality of tanks (5) in a usage position under said build platform (3) is done by rotating said carrousel (6) and then raising said tank (5) to said usage position.

7. Device (1) according to any one of the claims 1 to 3, wherein said tank positioning means is configured to change the position of said build platform (3).

8. Device (1) according to any one of the claims 1 to 7, wherein said tank positioning means further comprises tank elevating means, configured to raise said tank (5) positioned under said build platform (3) to said usage position and to lower said tank (5) from said usage position to a free position wherein said tank (5) does not contact said build platform (3) and can be moved away from said build platform (3).

9. Device (1) according to claim 8, wherein said tank elevating means further comprises at least one cam (9) configured to push said tank (5) positioned under said build platform (3) in a vertical direction.

10. Device (1) according to any one of the claims 1 to 9, wherein each cleaning tank (52) of said at least one cleaning tank (52) is provided with an idle roller (521) horizontally arranged over a cleaning product repository, wherein, in said usage position, said idle roller (521) is parallel to said build platform (3) and contacts said build surface (4) of said build platform (3), such that a rotation of said build platform (3) drives said idle roller (521) to rotate and said cleaning product is transferred from said repository to said build surface (4) by means of said idle roller (521).

11. Device (1) according to any one of the claims 1 to 10, further comprising resin temperature control means, configured to control, at least, the temperature of a resin contained in one resin tank (51) of said plurality of resin tanks (51), preferably at least when said resin tank (51) is in said usage position, more preferably wherein said resin temperature control means is configured to control the temperature of each resin contained in each resin tank (51) of said plurality of resin tanks (51).

12. Device (1) according to any claim 11, wherein said resin temperature control means comprises heating means, preferably comprising at least one of a resistance and a thermoelectric cell, more preferably also comprising cooling means, even more preferably comprising a thermoelectric cell.

13. 3D printing method for manufacturing at least one tubular 3D object (2), in particular a stent, each 3D object (2) manufactured by 3D printing according to a printing model; said method making use of a 3D printing device (1) according to any one of the claims 1 to 12, wherein said method is executed by said control means by controlling said laser source (10), said beam positioning means (11), said rotation control means (7), and said tank positioning means according to said printing model.

14. Tubular 3D object (2) obtainable by the 3D printing method according to claim 19, wherein said tubular 3D object (2) is made of at least two different resins, and wherein, in at least one point of said tubular 3D object (2), at least two of said different resins are welded together forming radial layers, wherein a layer made of a first resin is under a layer made of a second resin, and wherein said layers are welded by the polymerization of said resins.

15. Tubular 3D object (2) according to claim 20, wherein said tubular 3D object (2) is a stent.
